# EUROPÄISCHE PATENTSCHRIFT

(11) **EP 3 821 854 B1**
(45) Veröffentlichungstag und Bekanntmachung des Hinweises auf die Patenterteilung: **07.01.2026**
(21) Anmeldenummer: 19208824.3
(22) Anmeldetag: 13.11.2019
(51) Int. Cl.: A61F 2/36, A61F 2/38, A61F 2/40, A61L 27/04, A61L 27/06, A61F 2/42, A61F 2/30, A61L 27/30, A61F 2/32

(54) **ZIRKONIUMBESCHICHTETE IMPLANTATKOMPONENTE UND DEREN VERWENDUNG**
ZIRCONIA-COATED IMPLANT COMPONENT AND USE THEREOF
COMPOSANT D'IMPLANT REVÊTU DE ZIRCONIUM ET SON UTILISATION

(43) Veröffentlichungstag der Anmeldung: 19.05.2021
(73) Patentinhaber: Waldemar Link GmbH & Co. KG, 22339 Hamburg (DE)
(72) Erfinder: CSASZAR, Richard, 23795 Bad Segeberg (DE); LINK, Helmut D., 22397 Hamburg (DE)
(74) Vertreter: AWA Denmark A/S

(56) Entgegenhaltungen:
- WO-A1-2018/189159
- DE-A1- 102014 206 151
- US-A- 5 861 042
- US-A1- 2016 278 928

## Beschreibung

### TECHNISCHES GEBIET

Die vorliegende Offenbarung betrifft eine Implantatkomponente mit einem Verbindungsabschnitt zum Verbinden mit einer weiteren Implantatkomponente, wobei die Implantatkomponente ein Prothesenschaft ist und wobei der Verbindungsabschnitt zumindest abschnittsweise mit einer Zr-Beschichtung beschichtet ist sowie ein Implantat mit zumindest einer Implantatkomponente. Die vorliegende Offenbarung betrifft ferner die Verwendung einer Zr-Beschichtung zur Vorbeugung gegen Korrosion.

### STAND DER TECHNIK

Endoprothesen, beispielsweise Hüftendoprothesen können einen modularen Aufbau aufweisen, d. h. sie sind aus zumindest zwei getrennt voneinander wählbaren Komponenten zusammengesetzt. Dieser Aufbau verleiht den Prothesen eine hohe Anpassungsfähigkeit an die individuellen Anforderungen eines Patienten.

Die individuellen Anforderungen eines Patienten können beispielsweise konkrete Abmaße, Geometrien, Werkstoffkombinationen und/oder Befestigungsmechanismen beinhalten. Die Verwendung modularer Prothesen erlaubt es, die Vielzahl an Anforderungen zu berücksichtigen, ohne dass Implantate für jede mögliche Permutation vorrätig gehalten oder individuell hergestellt werden müssen.

Einzelne Implantatkomponenten einer modular aufgebauten Endoprothese können über Verbindungsabschnitte zu einer Endoprothese bzw. einem Implantat zusammengesetzt werden. Hierbei haben sich vor allem Konusverbindungen bewährt. Eine Konusverbindung kann beispielsweise einen im Wesentlichen kegelförmig ausgebildeten Vorsprung (männlicher Konus) und eine Öffnung mit einem sich verjüngenden Querschnitt (weiblicher Konus) aufweisen. Der männliche Konus und der weibliche Konus weisen jeweils eine Konusachse auf, die während des Einführens des Vorsprungs in die Öffnung (d.h., während des Verbindens der Implantatkomponenten) zusammenfallen. Die Konusverbindung ist somit auch selbstzentrierend.

Wenn der männliche Konus in den weiblichen Konus eingebracht ist, und die Implantatkomponenten in Richtung der Konusachse mit einer Kraft beaufschlagt werden, führt dies zu einer bezüglich der Konusachse radial orientierten elastischen Aufweitung des weiblichen Konus und zu einer Kompression des männlichen Konus. Die aus Aufweitung und Kompression resultierenden Rückfederkräfte wiederum erzeugen eine Klemmkraft, die bei Selbsthemmung zu einer reibschlüssigen Verbindung der Implantatkomponenten führt.

Solche Konusverbindungen werden beispielsweise bei Hüftendoprothesen vorgesehen. Einfach modulare Hüftendoprothesen weisen eine derartige Verbindung beispielsweise zwischen einem Prothesenschaft und einem Hüftkopf (Gelenkkopf) auf. Ferner sind mehrfach modulare Hüftendoprothesen bekannt, die ein Zwischenstück aufweisen, das zwischen dem Prothesenschaft und dem Hüftkopf angeordnet ist, um eine bessere Anpassung der Endoprothese an die Anatomie des Patienten zu ermöglichen. In diesem Fall können beispielsweise der Prothesenschaft und das Zwischenstück und/oder das Zwischenstück und der Hüftkopf mit einer zuvor beschriebenen Konusverbindung verbunden werden.

Es hat sich jedoch gezeigt, dass bei den oben beschriebenen modularen Endoprothesen Spaltkorrosion und/oder Reibkorrosion in der Konusverbindung auftreten kann (Fachbegriff: fretting bzw. fretting corrosion). Insbesondere betrifft diese Erscheinung die Kontaktflächen der Implantatkomponenten, also die Mantelfläche des männlichen Konus und die innere Umfangsfläche des weiblichen Konus.

Derartige Korrosionserscheinungen können mitunter zu einem fatalen Versagen des Implantats führen. Dieses Versagen tritt insbesondere als Abbrechen des männlichen Konus auf. Als Ursache für die Korrosion werden mitunter Mikrobewegungen zwischen den Verbindungsflächen zweier Implantatkomponenten sowie die daraus resultierenden Spannungen verantwortlich gemacht. Die Korrosion kann sich mitunter in Form von Rissen und/oder Abrieb einer Passivschicht auf der Oberfläche des Implantatmaterials äußern. Dabei wird angemerkt, dass Korrosionserscheinungen nicht nur bei Metall-Metall-Paarungen auftreten können, sondern auch bei Metall-Keramik-Paarungen, wie beispielsweise bei Hüftendoprothesen mit Metallschaft und Keramikkopf.

Weiterhin wird davon ausgegangen, dass derartige Konusverbindungen zwar nach dem oben beschriebenen Prinzip funktionieren, die Verbindung jedoch fertigungsbedingt nicht über die gesamte Länge des Konus stattfindet. So wurde festgestellt, dass die Konusverbindung vor allem im proximalen Bereich, d. h. am Ende des zulaufenden Konus, zustande kommt. Die Folge sind höhere Spannungen an dessen Oberfläche, welche die oben beschriebenen Korrosionserscheinungen begünstigen. Verstärkt wird dieser Effekt zudem dadurch, dass Konusverbindungen im Regelfall spanend, insbesondere durch Drehen, gefertigt werden. Dabei entsteht eine hierfür charakteristische wellenförmige Oberfläche im Mikrometerbereich. Auch dies kann Spannungsspitzen und lokale Verformungen bei einer Konusverbindung hervorrufen.

Neben dem oben beschriebenen Materialversagen führen die vorstehend beschriebenen Korrosionserscheinungen zudem regelmäßig zur Freisetzung von Metallionen, Metalloxiden, Metallorganophosphaten und/oder von kleinen Metallpartikeln, die wiederum die mechanischen Abriebserscheinungen verstärken.

Dadurch können bei Patienten Schmerzen, eine aseptische Lockerung der Endoprothese und/oder negative Folgewirkungen für das umliegende Gewebe auftreten. So kann insbesondere eine Metallose entstehen, d. h. ein unnatürliches Vorkommen von Abriebpartikeln im Gewebe. Eine mögliche Folge hiervon ist das Entstehen sogenannter Pseudotumore. Auch sind allergischen Reaktionen möglich, die ebenfalls eine Revision der Prothese erforderlich machen können.

Um den vorstehend beschriebenen Problemen entgegenzuwirken, wurden bisher mitunter konstruktive und fertigungstechnische Maßnahmen ergriffen. Beispielsweise wurde angestrebt, dass Maß der Korrosionserscheinungen durch präzisiere Herstellungsverfahren oder durch angepasste Kontaktflächengeometrien zu reduzieren. Aus der DE 102014206151 A1 ist zudem eine Konusverbindung mit einer TiNb-Beschichtung bekannt, die gegenüber unbeschichteten Verbindungen eine geringere Korrosionsanfälligkeit aufweist.

Aus der Druckschrift US 2016/278928 A1 is eine Prothese bekannt, die folgendes umfasst: Einen Femoraler Schaft mit einem kegelstumpfförmigen Oberschenkelhals, einen Femoraler Kopf mit einer Gelenkauflagefläche mit einem Außendurchmesser von 26 mm oder mehr und einer kegelstumpfförmig zulaufenden Innenausnehmung; und eine Hülse, die einen kegelstumpfförmigen Körper zum Einsetzen in die Aussparung des Femoraler Kopfes und eine kegelstumpfförmige innere Aussparung zur Aufnahme des kegelstumpfförmigen Femoraler Schaftes umfasst. Eine Oberfläche der Aussparung der Hülse besteht aus oxidiertem Zirkonium oder einer oxidierten Zirkoniumlegierung, um mechanisch unterstützter Spaltkorrosion zu widerstehen oder diese zu minimieren.

Als weitere Möglichkeit wurde eine Verwendung von Nitridbeschichtungen in Erwägung gezogen, um die Abriebeigenschaften von Implantatkomponenten zu verbessern. Diese werden zum Beispiel für die Laufflächen von Endoprothesen verwendet. Es hat sich jedoch herausgestellt, dass dort andere Abnutzungsbedingungen vorherrschen als in einem Verbindungsabschnitt. Dies mag ein Grund dafür sein, dass Nitridbeschichtungen hier zu keinen zufriedenstellenden Ergebnissen geführt haben.

Da also in Verbindungsabschnitten mit oder ohne Beschichtung weder ein frühzeitiges Versagen noch Wechselwirkungen mit Abriebmaterial ausgeschlossen werden können, besteht weiterhin das Bestreben, die Korrosionserscheinungen über das bisher erreichte Maß hinaus weiter zu reduzieren.

### ZUSAMMENFASSUNG DER ERFINDUNG

Dem Gegenstand der vorliegenden Offenbarung lag die Aufgabe zugrunde, den vorstehend beschriebenen Problemen entgegenzuwirken und Verbesserungen bei einer Implantatkomponente mit einem Verbindungsabschnitt und insbesondere Verbesserungen bezüglich Spaltkorrosion und/oder Reibkorrosion (fretting corrosion) zu erwirken. Diese Aufgabe wird durch eine Implantatkomponente nach Anspruch 1, eine modulare Endoprothese nach Anspruch 10, oder eine Verwendung nach Anspruch 13 gelöst. Bevorzugte Ausführungsformen sind in den Unteransprüchen angegeben.

Eine Implantatkomponente gemäß der vorliegenden Offenbarung weist zumindest einen Verbindungsabschnitt auf, der zumindest abschnittsweise mit einer Zr-Beschichtung beschichtet ist. Bevorzugt ist der gesamte Verbindungsabschnitt mit einer Zr-Beschichtung beschichtet. Besonders bevorzugt sind zumindest diejenigen Flächen des Verbindungsabschnitts, die dazu ausgebildet sind, mit Flächen eines Verbindungsabschnittsgegenstücks einer anderen Implantatkomponente in Kontakt zu treten, mit einer Zr-Beschichtung beschichtet.

Die Zr-Beschichtung weist eine Dicke zwischen 1 µm und 20 µm, bevorzugt zwischen 1 µm und 6 µm auf. Bei Zr handelt es sich im Rahmen der vorliegenden Offenbarung um elementares Zirconium (Element mit der Ordnungszahl 40 im Periodensystem), nicht jedoch um Zirkon (Zr[SiO₄]) oder andere zirconiumbasierte Minerale oder Keramiken. Zirconiumbasierte Oxide, die sich an der Oberfläche einer Zr-Beschichtung in Form einer Passivschicht ausbilden können, werden einer Zr-Beschichtung gemäß der vorliegenden Offenbarung zugeordnet.

Die vorstehend beschriebene Implantatkomponente kann mit einer gegenüber dem Stand der Technik signifikant verringerten Anfälligkeit für Spaltkorrosion und/oder Reibkorrosion im Bereich des Verbindungsabschnitts in Verbindung gebracht werden. Diese Eigenschaft ist im Wesentlichen auf die Wahl des Beschichtungsmaterials (Zr) in Kombination mit der Schichtdicke zurückzuführen.

Mit dem weiter unten beschriebenen Messverfahren wurde gezeigt, dass ein Verbindungsabschnitt mit einer Zr-Beschichtung (mit einer offenbarungsgemäßen Schichtdicke) eine signifikant geringere Anfälligkeit für Spaltkorrosion und/oder Reibkorrosion (fretting corrosion) aufweist als beispielsweise ein Verbindungsabschnitt mit Titan-Niob (TiNb) oder ein Verbindungsabschnitt, der mit einer Kobaltbasislegierung(z.B. CoCrMo) ausgebildet ist.

Durch die geringere Anfälligkeit für Spaltkorrosion und/oder Reibkorrosion (fretting corrosion) wird angenommen, dass die Passivschicht an der Oberfläche der Zr-Beschichtung wesentlich stabiler ist als die Passivschicht anderer metallischer Beschichtungen oder Werkstoffe. Zudem sprechen bisherige Untersuchungen für ein hohes Maß an Biokompatibilität, was insbesondere gegen allergische Reaktionen vorbeugt.

Weiterhin wird davon ausgegangen, dass die geringe Anfälligkeit der Zr-Beschichtung für Korrosion auf die mechanischen Eigenschaften dieser Beschichtung zurückzuführen ist. Insbesondere besitzt die Beschichtung eine ausreichende Duktilität. Als Folge hiervon wird ein gleichmäßigerer Kontakt beim Verbindungsabschnitt ermöglicht und somit Spannungsspitzen abgebaut. Dies ist zum Beispiel bei der oben erwähnten, fertigungsbedingten wellenförmigen Oberflächenstruktur von Vorteil.

Die Schichtdicke der Zr-Beschichtung einer Implantatkomponente gemäß der vorliegenden Offenbarung liegt bevorzugt zwischen 3 µm und 6 µm, und besonders bevorzugt zwischen 3 µm und 5 µm.

Ziel ist es, die Dicke der Schicht so hoch zu wählen, dass eine den mechanischen Belastungen widerstehende Beschichtung erzielt wird. Gleichzeitig ist anzustreben, die Beschichtung nicht zu stark zu wählen, damit ein stabiler Reibschluss zwischen den Flächen des Verbindungsbereichs erreicht wird.

Die Herstellung einer Zr-Schicht mit einer großen Dicke ist mit höheren Kosten verbunden. Bei einer zu dünnen Schicht kann hingegen der angestrebte Effekt (Verringerung der Anfälligkeit für Spaltkorrosion und/oder Reibkorrosion) gegebenenfalls nicht dauerhaft und reproduzierbar erzielt werden. Die oben aufgeführten, (besonders) bevorzugten Schichtdicken sind mit vertretbar geringen Herstellungskosten verbunden und erlauben es gleichzeitig, die Anfälligkeit des Verbindungsabschnitts für Spaltkorrosion und/oder Reibkorrosion gegenüber bekannten Beschichtungen zu reduzieren. Die oben genannten Schichtdicken können somit auch als Lösung eines mehrdimensionalen Optimierungsproblems hinsichtlich Herstellungskosten und Eintritt des angestrebten Effekts angesehen werden.

Eine Implantatkomponente gemäß der vorliegenden Offenbarung kann einen Verbindungsabschnitt aufweisen, der mit einem weiblichen und/oder männlichen Konus gebildet ist. Bevorzugt ist der weibliche und/oder männliche Konus rotationssymmetrisch.

Eine Konusverbindung kann durch ihre verkeilenden Eigenschaften als selbststabilisierend angesehen werden. Zudem zeichnet sie sich durch verhältnismäßig geringe Fertigungskosten aus und erlaubt eine präzise Fertigung der Passflächen (beispielsweise mit einer Rundlauftoleranz unter 0,1 mm, unter 0,05 mm oder unter 0,01 mm). Als Folge der hohen Fertigungspräzision, die bei derartigen Geometrien erreicht werden kann, kann der Betrag des freien Volumens zwischen Passflächen auf ein Minimum reduziert werden. Dadurch werden die über die Konusverbindung übertragenen Kräfte gleichmäßig abgetragen. Spannungsspitzen werden verhindert.

Wenn die Konusverbindung rotationssymmetrisch ausgebildet ist, steht dem operierenden Chirurgen während des Einsetzens des Implantats ein weiterer Freiheitsgrad zum Ausrichten des Implantats zur Verfügung, nämlich ein Drehfreiheitsgrad um die Konusachse. Dieser Drehfreiheitsgrad erlaubt es bei bestimmten Implantaten, die Implantatgeometrie schnell und einfach an die Patientengeometrie anzupassen.

Alternativ zur Konusverbindung kann der Verbindungsabschnitt beispielswese als achsparalleler Zylinder und/oder als achsparallele Bohrung ausgebildet sein. Der achsparallele Zylinder und/oder die achsparallele Bohrung kann, wenn der Verbindungsabschnitt mit einer anderen Implantatkomponente verbunden ist, teil eines Passsystems sein, beispielsweise mit einer Presspassung oder einer Übergangspassung (z.B. H7p6 oder H7n6).

Der Verbindungsabschnitt kann ferner einen Anschlag mit einer Anschlagfläche aufweisen, wobei die Anschlagfläche beispielsweise im Wesentlichen senkrecht auf der Konus- bzw. Zylinderachse stehen kann oder einen Winkel mit dieser einschließen kann. Die Anschlagfläche ist bevorzugt im Wesentlichen rotationssymmetrisch bezüglich der Konus- bzw. Zylinderachse ausgebildet.

Eine Implantatkomponente gemäß der vorliegenden Offenbarung kann eine Metalllegierung, beispielsweise eine Titanbasislegierung oder eine Kobaltbasislegierung, aufweisen. Bevorzugt ist die Implantatkomponente im Wesentlichen aus einer Metalllegierung, beispielsweise einer Titanbasislegierung oder einer Kobaltbasislegierung ausgebildet. Mit anderen Worten ist die Zr-Beschichtung bevorzugt auf einen Verbindungsabschnitt einer im Wesentlichen aus einer der vorstehend genannten Legierungen gebildeten Implantatkomponente aufgebracht. Insbesondere kann eine offenbarungsgemäße Implantatkomponente eine CoCr-Gusslegierung aufweisen, die beispielsweise ca. 62-66 Gew.-% Kobalt, ca. 27-31 Gew.-% Chrom und 4-5 Gew.-% Molybdän enthält. Eine derartige Legierung kann in geringen Mengen jedoch auch Kohlenstoff, Silizium, Mangan, Eisen und/oder weitere Begleitelemente enthalten (z. B. gemäß ISO 5832-4, ASTM F75). Weitere Beispiele von Metalllegierungen, die bei einer Implantatkomponente zum Einsatz kommen können, sind CoCr-Schmiedelegierungen (z. B. gemäß ISO 5832-12), Stahllegierungen (z. B. gemäß ISO 5832-1) oder Titanlegierungen (z. B. gemäß ISO 5832-3 oder ISO 5832-11).

Die Implantatkomponente kann jedoch auch andere Werkstoffe, beispielsweise eine Keramik oder eine Kunststoffsorte aufweisen, oder im Wesentlichen aus einem oder mehreren dieser Werkstoffe gebildet sein. Als Keramikwerkstoffe können beispielsweise Aluminium-, Titan-, Zirconium- und/oder Magnesiumbasierte Keramiken verwendet werden. Als Kunststoffsorten kommen beispielsweise UHMW-PE, PP, PEEK oder POM in Frage.

Die genannten metallischen Werkstoffe bieten beispielsweise eine hohe mechanische Festigkeit in Verbindung mit ausgezeichneten Eigenschaften bezüglich Biokompatibilität. Zudem sind insbesondere Titanlegierungen für ein ausgezeichnetes Einwachsverhalten von Knochengewebe bekannt. Die genannten Keramikwerkstoffe können Vorteile bezüglich der Dauerfestigkeit aufweisen. Die obigen Kunststoffsorten können vorteilhafte Gleiteigenschaften, eine vorteilhafte Schlagzähigkeit, eine vorteilhaft hohe Duktilität und/oder ausgezeichnete gewichtsbezogene Festigkeitseigenschaften aufweisen.

Eine Implantatkomponente gemäß der vorliegenden Offenbarung kann beispielsweise ein Prothesenschaft, ein Zwischenstück oder eine Gelenkkomponente, insbesondere ein Gelenkkopf, sein. Die Implantatkomponente kann beispielsweise dazu ausgebildet sein, als Bestandteil einer Hüftgelenkersatzprothese, einer Kniegelenkersatzprothese, einer Ellenbogengelenkersatzprothese, einer Schultergelenkersatzprothese oder einer Fuß-, Hand,- oder Fingergelenkersatzprothese eingesetzt zu werden.

Bei einer offenbarungsgemäßen Implantatkomponente kann die Zr-Beschichtung beispielsweise einen Zr-Anteil von zumindest 90 At.-%, aufweisen. Bevorzugt weist die Zr-Beschichtung einen Zr-Anteil von zumindest 94 At.-% und besonders bevorzugt von zumindest 99,5 At-% auf. Die Angaben sind als Stoffmengenanteile zu verstehen. Bei einer hohen Reinheit der Beschichtung, beispielsweise bei den oben genannten Zr-Anteilen, kann eine Verringerung der Anfälligkeit für Spaltkorrosion und/oder Reibkorrosion besonders gut ausgeprägt sein.

Bei einer offenbarungsgemäßen Implantatkomponente kann die Beschichtung durch ein physikalisches Gasphasenabscheidungsverfahren oder durch ein Galvanisierungsverfahren aufgebracht sein. Ein Galvanisierungsverfahren kann gegenüber anderen Verfahren Kostenvorteile aufweisen. Ein Gasphasenabscheidungsverfahren (z.B. PVD-Verfahren) zeichnet sich durch eine hohe Genauigkeit bei der Einhaltung der angestrebten Schichtdicke aus. Beispielsweise können mit einem PVD-Verfahren Abweichungen von nicht mehr als ±20% (bezogen auf eine Sollschichtdicke) erreicht werden. Insbesondere können mit Gasphasenabscheidungsverfahren auch bei komplexen Geometrien sehr gleichförmige Schichtdicken erzeugt werden. Eine Beschichtung, die mit einem Gasphasenabscheidungsverfahren aufgebracht ist, haftet zudem wesentlich besser an der Oberfläche des beschichteten Werkstücks als eine Beschichtung, die mit einem anderen Verfahren aufgebracht worden ist.

Gemäß der vorliegenden Offenbarung wird ferner eine modulare Endoprothese bereitgestellt, die zumindest eine, bevorzugt jedoch genau eine der oben beschriebenen offenbarungsgemäßen Implantatkomponenten aufweist. Bevorzugt weist die offenbarungsgemäße modulare Endoprothese ferner eine zweite Implantatkomponente mit einem Verbindungsabschnittgegenstück auf, wobei das Verbindungsabschnittgegenstück dazu ausgebildet ist, mit dem Verbindungsabschnitt der offenbarungsgemäßen Implantatkomponente in Eingriff zu gehen. In anderen Worten sind der Verbindungsabschnitt und das Verbindungsabschnittgegenstück komplementär ausgeführt. Das Verbindungsabschnittgegenstück weist vorzugsweise keine Zr-Beschichtung auf.

Es hat sich gezeigt, dass eine Verringerung der Anfälligkeit für Spaltkorrosion und/oder Reibkorrosion bereits eintritt, wenn der Verbindungsabschnitt einer ersten Implantatkomponente einer modularen Endoprothese eine offenbarungsgemäße Zr-Beschichtung aufweist, während ein mit dem Verbindungsabschnitt verbundenes Verbindungsabschnittgegenstück einer zweiten Implantatkomponente keine Zr-Beschichtung aufweist. Jedoch können bei einer offenbarungsgemäßen modularen Endoprothese auch sowohl der Verbindungsabschnitt, als auch das mit dem Verbindungsabschnitt verbundene Verbindungsabschnittgegenstück einer zweiten Implantatkomponente eine Zr-Beschichtung aufweisen.

Bei einer offenbarungsgemäßen modularen Endoprothese kann die erste Implantatkomponente beispielsweise ein Prothesenschaft sein, bei dem der Verbindungsabschnitt ein männlicher Konus ist, und die zweite Implantatkomponente kann beispielsweise eine Gelenkkomponente, insbesondere ein Gelenkkopf sein, bei dem das Verbindungsabschnittgegenstück als weiblicher Konus ausgeführt ist.

Zudem wird auch die Verwendung einer Zr-Beschichtung zur Vorbeugung gegen Korrosion an einem Verbindungsabschnitt einer Implantatkomponente offenbart, wobei die Beschichtung eine Dicke von 1-20 µm, bevorzugt 1-6 µm und besonders bevorzugt 3-5 µm aufweist, und wobei die Implantatkomponente bevorzugt eine der vorstehend beschriebenen offenbarungsgemäßen Implantatkomponenten ist. Diese Verwendung weist die Gleichen oder vergleichbare Vorteile bzw. Effekte auf wie eine offenbarungsgemäße Implantatkomponente bzw. eine offenbarungsgemäße modulare Endoprothese.

### KURZE BESCHEIRUNG DER ZEICHNUNGEN

- Fig. 1: zeigt eine Ausführungsform einer modularen Endoprothese gemäß der vorliegenden Offenbarung im zerlegten Zustand;
- Fig. 2: zeigt die Ausführungsform der modularen Endoprothese aus Fig. 1 in einem verbundenen Zustand.
- Fig. 3a: zeigt den Verlauf der Stromstärke, die bei zyklischer Belastung einer Endoprothese mit einer bekannten Implantatkomponente gemessen wurde;
- Fig. 3b: zeigt den Verlauf der Stromstärke, die bei zyklischer Belastung einer Endoprothese mit einer Implantatkomponente gemäß der vorliegenden Offenbarung gemessen wurde.

### AUSFÜHRLICHE BESCHREIBUNG BEVORZUGTER AUSFÜHRUNGSFORMEN

Die nachfolgend beschriebenen bevorzugten Ausführungsformen stellen lediglich Beispiele dar und sind nicht als beschränkend anzusehen. Gleiche Bezugszeichen, die in verschiedenen Figuren aufgeführt sind, benennen identische, einander entsprechende, oder funktionell ähnliche Elemente.

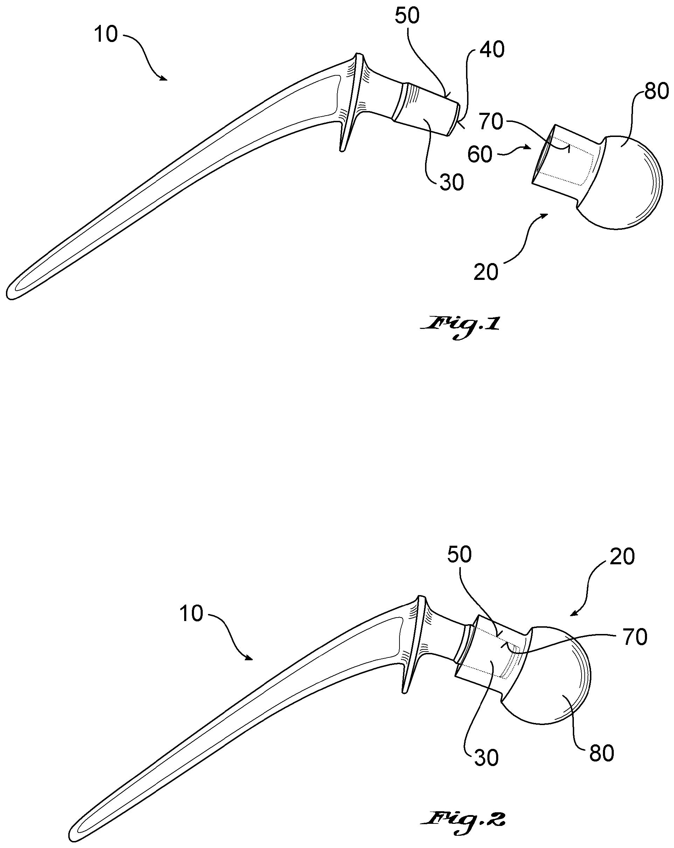

Fig. 1 zeigt eine Ausführungsform einer modularen Endoprothese gemäß der vorliegenden Offenbarung in einem nichtverbundenen Zustand. Die modulare Endoprothese weist als erste Implantatkomponente einen Prothesenschaft (10), und als zweite Implantatkomponente einen Gelenkkopf (20) auf. Der Prothesenschaft (10) weist einen Verbindungsabschnitt auf, der als männlicher Konus (30) ausgebildet ist. Der männliche Konus (30) weist eine Stirnfläche (40) und eine äußere Umfangsfläche (50) auf. Der männliche Konus (30) ist zumindest abschnittsweise mit einer Zr-Beschichtung beschichtet, wobei die Zr-Beschichtung eine Schichtdicke von 1-20 µm, bevorzugt 1-6 µm aufweist. Bevorzugt ist zumindest die gesamte äußere Umfangsfläche (50) des männlichen Konus (30) mit einer Zr-Beschichtung beschichtet. Ferner kann zusätzlich die Stirnfläche (40) des männlichen Konus (30) mit einer Zr-Beschichtung beschichtet sein. Der Übergang von der äußeren Umfangsfläche (50) des männlichen Konus (30) hin zur Stirnfläche (40) des männlichen Konus (30) kann beispielsweise als Fase oder Kantenverrundung ausgebildet sein. Auch dieser Übergang (d.h., die Fase bzw. Kantenverrundung) kann eine offenbarungsgemäße Zr-Beschichtung aufweisen.

Der Gelenkkopf (20) weist ein Verbindungsabschnittgegenstück auf, das als weiblicher Konus (60) ausgebildet ist. Der weibliche Konus (60) weist eine innere Umfangsfläche (70) und eine Bodenfläche auf. Der Gelenkkopf (20) weist ferner eine Gelenkkugel (80) auf. Die Gelenkkugel ist bevorzugt als Kugelsegment mit einer im Wesentlichen sphärischen Oberfläche ausgebildet, die als Gelenkfläche dieser Gelenkkomponente dient. In den Figuren 1 und 2 ist der weibliche Konus (60) in einem der Gelenkkugel im Wesentlichen gegenüberliegenden Anschlussbereich ausgebildet. Der Anschlussbereich mit dem darin ausgebildeten weiblichen Konus (60) steht von der Seite der Gelenkkugel hervor, welcher das Kugelsegment begrenzt. Der weibliche Konus (60) kann sich dabei bis in den Bereich des Kugelsegments erstrecken. Ebenso ist es möglich, dass der Anschlussbereich nicht als Vorsprung, sondern als Fläche ausgebildet ist, die das Kugelsegment und damit die Gelenkfläche definiert. In diesem Fall ist der weibliche Konus (60) innerhalb des Kugelsegments ausgebildet.

In der vorliegenden Ausführungsform weist weder die innere Umfangsfläche (70), noch die Bodenfläche des weiblichen Konus (60) eine Zr-Beschichtung auf. In einer Abwandlung der oben beschriebenen Ausführungsform kann jedoch auch der weibliche Konus (60) des Gelenkkopfs (20) eine Zr-Beschichtung aufweisen, während der männliche Konus (30) des Prothesenschafts (10) keine Zr-Beschichtung aufweist. Gemäß einer weiteren Modifikation können sowohl der weibliche Konus (60) des Gelenkkopfs (20) als auch der männliche Konus (30) des Prothesenschafts (10) eine Zr-Beschichtung aufweisen. Bevorzugt ist zumindest die gesamte äußere Umfangsfläche (50) des männlichen Konus (30) oder die gesamte innere Umfangsfläche (70) des weiblichen Konus (60) mit einer Zr-Beschichtung beschichtet.

In einer nicht in den Figuren 1 und 2 dargestellten Variante der oben beschrieben Ausführungsform können der weibliche Konus (60) und der männliche Konus (30) auch vertauscht sein. So kann beispielsweise auch der Prothesenschaft (10) einen weiblichen Konus aufweisen, und der Gelenkkopf (20) kann einen männlichen Konus aufweisen.

In Fig. 2 ist die oben beschriebene Ausführungsform der offenbarungsgemäßen modularen Endoprothese in einem verbundenen Zustand dargestellt. Aus Fig. 2 geht hervor, dass die innere Umfangsfläche (70) des weiblichen Konus (60) im verbundenen Zustand mit der äußeren Umfangsfläche (50) des männlichen Konus (30) in Kontakt steht. Um eine Doppelpassung (die einem Verkeilungseffekt des männlichen Konus (30) und des weiblichen Konus (60) ggf. entgegenstehen könnte) zu vermeiden, ist es bevorzugt, dass die Geometrie des männlichen Konus (30) und die Geometrie des weiblichen Konus (60) derart aufeinander abgestimmt sind, dass die Stirnfläche (40) des männlichen Konus (30) und die Bodenfläche des weiblichen Konus (60) nicht in Kontakt stehen.

Wenn die Stirnfläche (40) des männlichen Konus (30) und die Bodenfläche des weiblichen Konus (60) (und/oder etwaige Fasen, Radien oder Übergangsbereiche) nicht miteinander in Kontakt stehen, ist davon auszugehen, dass an diesen Flächen keine Spaltkorrosion und/oder Reibkorrosion auftritt. Somit ist eine Zr-Beschichtung an diesen Flächen nicht notwendig. Dennoch kann an diesen Flächen eine Zr-Beschichtung vorgesehen sein. Insbesondere kann es aus anderen Gründen vorteilhaft sein, an der Stirnfläche (40) des männlichen Konus (30), an der Bodenfläche des weiblichen Konus (60) und/oder an etwaigen Fasen, Radien oder Übergangsbereichen eine Zr-Beschichtung vorzusehen. Wenn auch in diesen Bereichen eine Zr-Beschichtung vorgesehen ist, kann dort beispielsweise ein Übergang von einer unbeschichteten Fläche zu einer beschichteten Fläche verhindert werden, wodurch wiederum das Risiko reduziert wird, dass Teile der beschichteten Fläche abplatzen (z.B. infolge einer Kerbwirkung und/oder infolge von Spannungsspitzen). Darüber hinaus entfällt (wenn auch die Stirnfläche (40) des männlichen Konus (30), die Bodenfläche des weiblichen Konus (60) und/oder etwaige Fasen, Radien oder Übergangsbereiche mitbeschichtet werden) die Notwendigkeit, diese Flächen während des Beschichtungsvorgangs zu maskieren. Dadurch können wiederum Kostenvorteile erzielt werden.

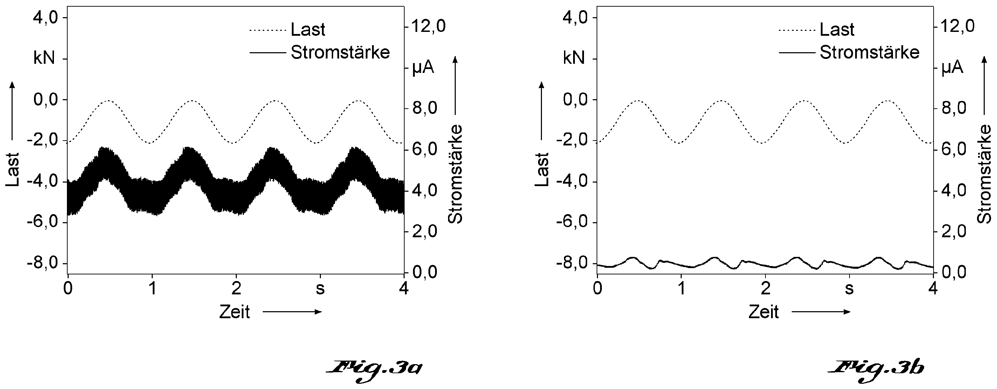

Die Figuren 3a und 3b veranschaulichen (auszugsweise) Messwerte einer experimentellen Untersuchung, bei der eine bekannte Implantatkomponente (Fig. 3a) und eine Implantatkomponente mit einer offenbarungsgemäßen Zr-Beschichtung (Fig. 3b) in Bezug auf das Auftreten von Spaltkorrosion und/oder Reibkorrosion untersucht wurden. Qualitative bzw. vergleichende Informationen über die Anfälligkeit eines Verbindungsabschnitts für Spaltkorrosion und/oder Reibkorrosion können als experimentelle Untersuchung beispielsweise mit einem Messverfahren nach ASTM F1875 - 98 (erneut zugelassen 2014) erlangt werden. Bei diesem Verfahren werden femorale Schaft- und Kopfkomponenten in ein Medium, beispielsweise eine Kochsalzlösung, eingebracht und mit einer zyklischen Last beaufschlagt. Die Schaft- und Kopfkomponenten sind mittels eines Verbindungsabschnitts verbunden. Ebenfalls in das Medium eingebracht werden Referenzelektroden, deren Beschichtungsmaterial mit dem Beschichtungsmaterial der zu prüfenden Schaft- und Kopfkomponenten übereinstimmt. Auch die Oberflächengröße der Referenzelektroden und die Oberflächengröße der (in das Medium eingebrachten Abschnitte der) Schaft- und Kopfkomponenten entsprechen einander.

Die Schaft- und Kopfkomponenten sowie die Referenzelektroden sind mit einem Strommessgerät verbunden, das es erlaubt, Ströme zu messen, die (über das Medium) zwischen den Schaft- und Kopfkomponenten einerseits und den Referenzelektroden andererseits fließen. Da die Oberfläche und das Beschichtungsmaterial der Schaft- und Kopfkomponenten und der Referenzelektroden identisch sind, sind diese Ströme jedoch nicht auf einen Potenzialunterschied zwischen den Schaft- und Kopfkomponenten und den Referenzelektroden zurückzuführen (Galvanische Zelle/Batterieeffekt). Vielmehr resultiert der messbare Stromfluss zwischen den Schaft- und Kopfkomponenten einerseits und den Referenzelektroden andererseits daraus, dass infolge der oben genannten zyklischen Krafteinwirkung Teile der Passivschicht (bzw. der Passivschichten) an der Oberfläche (bzw. den Oberflächen) des Verbindungsabschnitts abgerieben werden und sich neu bilden.

Aus den Messwerten bezüglich des Stromflusses zwischen den Schaft- und Kopfkomponenten und den Referenzelektroden können die im zeitlichen Verlauf gemittelte Stromstärke Iₘ, und die mittlere dynamische Stromstärke I_{d} bestimmt werden. Bei der mittleren dynamischen Stromstärke I_{d} handelt es sich um die Differenz aus der in einem bestimmten Zeitintervall gemessenen maximalen Stromstärke Iₘₐₓ und der minimalen Stromstärke Iₘᵢₙ (d.h., im Zeitintervall Δt1 gilt: I_{d,Δt1} = I_{max,Δt1} - I_{min,Δt1}).

Wenn bei einer ersten Kombination aus Schaft- und Kopfkomponenten ein geringerer Wert Iₘ gemessen wird als bei einer zweiten Kombination aus Schaft- und Kopfkomponenten, gilt dies als indirekter Nachweis dafür, dass die erste Kombination aus Schaft- und Kopfkomponenten weniger anfällig für Spaltkorrosion und/oder Reibkorrosion ist, als die zweite Kombination aus Schaft- und Kopfkomponenten.

Insbesondere gilt, wenn bei einer ersten Kombination aus Schaft- und Kopfkomponenten ein geringerer Wert Iₘ und ein geringerer Wert I_{d} gemessen wird als bei einer zweiten Kombination aus Schaft- und Kopfkomponenten, dies als indirekter Nachweis dafür, dass die erste Kombination aus Schaft- und Kopfkomponenten signifikant weniger anfällig für Spaltkorrosion und/oder Reibkorrosion ist als die zweite Kombination aus Schaft- und Kopfkomponenten.

Die bekannte Implantatkomponente der Fig. 3a und die Implantatkomponente mit offenbarungsgemäßer Zr-Beschichtung der Fig. 3b waren jeweils mit einem Verbindungsabschnitt mit einer weiteren Implantatkomponente verbunden, wodurch jeweils eine Endoprothese gebildet wurde. Die Geometrie der Endoprothese mit der bekannten Implantatkomponente, und die Geometrie der Endoprothese mit der Implantatkomponente mit offenbarungsgemäßer Zr-Beschichtung waren identisch. Ferner wurden bei beiden Endoprothesen identische Versuchsparameter gewählt. Wie aus den Figuren 3a und 3b hervorgeht, wurden die Endoprothesen mit einer zyklischen Last beaufschlagt, deren Frequenz 1 Hz betrug. Der Betrag der Last verlief periodisch zwischen 0,04 kN und 2,04 kN. In den Diagrammen in Fig. 3a und Fig. 3b ist die Last (in Kilonewton) auf der jeweils linken vertikalen Achse aufgetragen, wobei das negative Vorzeichen der Last auf deren Orientierung zurückzuführen ist (Drucklast). Auf der jeweils horizontalen Achse der Diagramme ist die Zeit in Sekunden aufgetragen. Auf der jeweils rechten vertikalen Achse ist die Stromstärke (in Microampere) aufgetragen, die während der zyklischen Belastung der Endoprothesen zwischen den Endoprothesen und den Referenzelektroden gemessen wurde.

Wie anhand von Fig. 3a nachzuvollziehen ist, wurden in einem bestimmten Zeitintervall bei der Endoprothese mit der bekannten Implantatkomponente eine im zeitlichen Verlauf gemittelte Stromstärke Iₘ = 4,49 µA, und eine mittlere dynamische Stromstärke I_{d} = 3,61 µmA gemessen. Wie anhand von Fig. 3b zu erkennen ist, wurden in einem bestimmten Zeitintervall bei der Endoprothese mit der Implantatkomponente mit der offenbarungsgemäßen Zr-Beschichtung eine im zeitlichen Verlauf gemittelte Stromstärke Iₘ = 0,49 µA, und eine mittlere dynamische Stromstärke I_{d} = 0,68 µmA gemessen. Ein Vergleich der Versuchsreihen lässt den Schluss zu, dass die Endoprothese mit der Implantatkomponente mit der offenbarungsgemäßen Zr-Beschichtung weniger anfällig für Spaltkorrosion und/oder Reibkorrosion ist als die Endoprothese mit der bekannten Implantatkomponente.

## Patentansprüche

1. Implantatkomponente (10), mit einem Verbindungsabschnitt (30), wobei der Verbindungsabschnitt (30) zumindest abschnittsweise mit einer Zr-Beschichtung beschichtet ist und die Beschichtung eine Dicke von 1-20 µm, bevorzugt 1-6 µm aufweist,
wobei die Zr-Beschichtung einen Zr-Anteil von zumindest 90 At.- % aufweist, und wobei
die Zr-Beschichtung zumindest eine von elementarem Zirconium und Zirconium-basierte Oxide umfasst, **dadurch gekennzeichnet, dass** die Implantatkomponente (10) ein Prothesenschaft (10) ist.

2. Implantatkomponente (10) nach Anspruch 1, bei der die Beschichtung eine Dicke von 3-6 µm, bevorzugt von 3-5 µm aufweist.

3. Implantatkomponente (10) nach einem der vorstehenden Ansprüche, bei welcher der Verbindungsabschnitt (30) einen weiblichen und/oder männlichen Konus aufweist.

4. Implantatkomponente (10) nach einem der vorstehenden Ansprüche, bei welcher der Verbindungsabschnitt (30, 60) rotationssymmetrisch ist.

5. Implantatkomponente (10) nach einem der vorstehenden Ansprüche, wobei die Implantatkomponente (10) eine Metalllegierung, bevorzugt eine Titanbasislegierung oder eine Kobaltbasislegierung, aufweist.

6. Implantatkomponente (10) nach einem der vorstehenden Ansprüche, wobei die Implantatkomponente (10) eine CoCr-Legierung aufweist.

7. Implantatkomponente (10) nach einem der vorstehenden Ansprüche, bei der die Zr-Beschichtung einen Zr-Anteil von zumindest 97 At.-% und bevorzugt zumindest 99,5 At.-% aufweist.

8. Implantatkomponente (10) nach einem der vorstehenden Ansprüche, bei der die Zr-Beschichtung eine physikalische aufgedampfte Zr-Beschichtung oder eine galvanische Zr-Beschichtung ist.

9. Modulare Endoprothese mit einer ersten Implantatkomponente (10) nach einem der vorstehenden Ansprüche.

10. Modulare Endoprothese nach Anspruch 9, die ferner eine zweite Implantatkomponente (20) mit einem Verbindungsabschnittgegenstück (60) aufweist, wobei das Verbindungsabschnittgegenstück (60) dazu ausgebildet ist, mit dem Verbindungsabschnitt (30) der ersten Implantatkomponente (10) in Eingriff zu gehen, wobei das Verbindungsabschnittgegenstück (60) vorzugsweise keine Zr-Beschichtung aufweist.

11. Modulare Endoprothese nach Anspruch 10, bei der die erste Implantatkomponente ein Prothesenschaft (10) mit einem als männlichem Konus ausgebildeten Verbindungsabschnitt (30) ist, und bei der die zweite Implantatkomponente ein Gelenkkopf (20) mit einem als weiblichen Konus ausgebildeten Verbindungsabschnittgegenstück (60) ausgebildet ist.

12. Verwendung einer Zr-Beschichtung zur Vorbeugung gegen Korrosion an einem Verbindungsabschnitt (30) einer Implantatkomponente (10), wobei die Beschichtung eine Dicke von 1-20 µm, bevorzugt 1-6 µm und besonders bevorzugt 3-5 µm aufweist,
**dadurch gekennzeichnet, dass** die Implantatkomponente (10) eine Implantatkomponente (10) nach einem der Ansprüche 1 bis 8 ist.

## Claims

1. An implant component (10) with a connecting section (30), wherein the connecting section (30) is coated at least in sections with a Zr coating and the coating has a thickness of 1-20 µm, preferably 1-6 µm, wherein the Zr coating has a Zr content of at least 90 at%, and wherein the Zr coating comprises at least one of elemental zirconium and zirconium-based oxides, **characterized in that** the implant component (10) is a prosthesis shaft (10).

2. The implant component (10) of claim 1, wherein the coating has a thickness of 3-6 µm, preferably 3-5 µm.

3. The implant component (10) of any one of the preceding claims, wherein the connecting section (30) has a female and/or a male cone.

4. The implant component (10) of any one of the preceding claims, wherein the connecting section (30, 60) is rotationally symmetrical.

5. The implant component (10) of any one of the preceding claims, wherein the implant component (10) comprises a metal alloy, preferably a titanium-based alloy or a cobalt-based alloy.

6. The implant component (10) of any one of the preceding claims, wherein the implant component (10) comprises a CoCr alloy.

7. The implant component (10) of any one of the preceding claims, wherein the Zr coating has a Zr content of at least 97 at% and preferably at least 99.5 at%.

8. The implant component (10) of any one of the preceding claims, wherein the Zr coating is a physical vapor-deposited Zr coating or a galvanic Zr coating.

9. A modular endoprosthesis with a first implant component (10) according to any one of the preceding claims.

10. The modular endoprosthesis of claim 9, further comprising a second implant component (20) with a connecting section counterpart (60), wherein the connecting section counterpart (60) is designed to engage with the connecting section (30) of the first implant component (10), wherein the connecting section counterpart (60) preferably does not have a Zr coating.

11. The modular endoprosthesis of claim 10, wherein the first implant component is a prosthesis shaft (10) with a connecting section (30) designed as a male cone, and wherein the second implant component is a joint head (20) with a connecting section counterpart (60) designed as a female cone.

12. A use of a Zr coating for preventing corrosion on a connecting section (30) of an implant component (10), wherein the coating has a thickness of 1-20 µm, preferably 1-6 µm and particularly preferably 3-5 µm,
**characterized in that** the implant component (10) is an implant component (10) of any one of claims 1 to 8.

## Revendications

1. Composant d'implant (10) avec une section de liaison (30), dans lequel la section de liaison (30) est revêtue au moins par section d'un revêtement en Zr et le revêtement présente une épaisseur de 1 à 20 µm, de préférence de 1 à 6 µm, dans lequel le revêtement en Zr présente une teneur en Zr d'au moins 90 % atomique, et dans lequel le revêtement de Zr comprend au moins l'un du zirconium élémentaire et des oxydes à base de zirconium, **caractérisé en ce que** le composant d'implant (10) est une tige de prothèse (10).

2. Composant d'implant (10) selon la revendication 1, dans lequel le revêtement a une épaisseur de 3 à 6 µm, de préférence de 3 à 5 µm.

3. Composant d'implant (10) selon l'une des revendications précédentes, dans lequel la section de liaison (30) présente un cône femelle et/ou mâle.

4. Composant d'implant (10) selon l'une des revendications précédentes, dans lequel la section de liaison (30, 60) est symétrique en rotation.

5. Composant d'implant (10) selon l'une des revendications précédentes, dans lequel le composant d'implant (10) présente un alliage métallique, de préférence un alliage à base de titane ou un alliage à base de cobalt.

6. Composant d'implant (10) selon l'une des revendications précédentes, dans lequel le composant d'implant (10) présente un alliage CoCr.

7. Composant d'implant (10) selon l'une des revendications précédentes, dans lequel le revêtement en Zr présente une teneur en Zr d'au moins 97 % atomiques et de préférence d'au moins 99,5 % atomiques.

8. Composant d'implant (10) selon l'une des revendications précédentes, dans lequel le revêtement en Zr est un revêtement en Zr déposé physiquement en phase vapeur ou un revêtement en Zr galvanique.

9. Endoprothèse modulaire avec un premier composant d'implant (10) selon l'une des revendications précédentes.

10. Endoprothèse modulaire selon la revendication 9, présentant en outre un second composant d'implant (20) avec une contrepartie de section de liaison (60), dans laquelle la contrepartie de section de liaison (60) est conçue pour venir en prise avec la section de liaison (30) du premier composant d'implant (10), dans laquelle la contrepartie de section de liaison (60) ne présente de préférence pas de revêtement en Zr.

11. Endoprothèse modulaire selon la revendication 10, dans laquelle le premier composant d'implant est une tige de prothèse (10) avec une section de liaison (30) conçue comme un cône mâle, et dans laquelle le second composant d'implant est une tête d'articulation (20) avec une contrepartie de section de liaison (60) conçue comme un cône femelle.

12. Utilisation d'un revêtement en Zr pour prévenir la corrosion sur une section de liaison (30) d'un composant d'implant (10), dans laquelle le revêtement présente une épaisseur de 1 à 20 µm, de préférence de 1 à 6 µm et, plus particulièrement, de 3 à 5 pm, **caractérisée en ce que** le composant d'implant (10) est un composant d'implant (10) selon l'une des revendications 1 à 8.
